(19) Europäisches Patentamt
European Patent Office
Office européen des brevets

(11) Veröffentlichungsnummer: **0 149 115**
**A2**

(12) **EUROPÄISCHE PATENTANMELDUNG**

(21) Anmeldenummer: **84114975.0**

(22) Anmeldetag: **08.12.84**

(51) Int. Cl.⁴: **C 07 D 409/14**
C 07 D 401/06, A 61 K 31/38
A 61 K 31/44

(30) Priorität: **17.12.83 DE 3345813**

(43) Veröffentlichungstag der Anmeldung:
**24.07.85 Patentblatt 85/30**

(84) Benannte Vertragsstaaten:
**AT BE CH DE FR GB IT LI LU NL SE**

(71) Anmelder: **BOEHRINGER INGELHEIM KG**
**D-6507 Ingelheim am Rhein(DE)**

(84) Benannte Vertragsstaaten:
**BE CH DE FR IT LI LU NL SE AT**

(71) Anmelder: **Boehringer Ingelheim International G.m.b.H**
**D-6507 Ingelheim am Rhein(DE)**

(84) Benannte Vertragsstaaten:
**GB**

(72) Erfinder: **Walther, Gerhard, Dr.**
**Pfarrer-Heberer-Strasse 37**
**D-6530 Bingen/Rhein(DE)**

(72) Erfinder: **Schneider, Claus, Dr.**
**Albrecht-Dürer-Strasse 19**
**D-6507 Ingelheim/Rhein(DE)**

(72) Erfinder: **Weber, Karl-Heinz, Dr.**
**Kaiser-Karl-Strasse 11**
**D-6535 Gau-Algesheim(DE)**

(72) Erfinder: **Böke-Kuhn, Karin, Dr.**
**Beethovenstrasse 11**
**D-6535 Gau-Algesheim(DE)**

(72) Erfinder: **Bechtel, Wolf Dietrich, Dr.**
**Mühlstrasse 3**
**D-6531 Appenheim(DE)**

(54) **Neue 2-(1-Imidazolyl)-ethanol-Derivate.**

(57) Neue 2-(1-Imidazolyl)-ethanol-Derivate der allgemeinen Formel

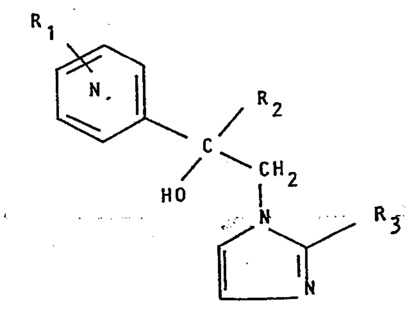

(I)

worin

$R_1$ Wasserstoff, eine Alkylgruppe mit 1 - 3 Kohlenstoffatomen oder ein Halogenatom;

$R_2$ einen der Reste

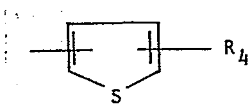

oder

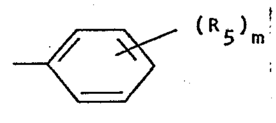

$R_3$ Wasserstoff oder den Methylrest;

$R_4$ Wasserstoff, einen Alkylrest mit 1 - 3 Kohlenstoffatomen oder ein Halogenatom;

$R_5$ Wasserstoff, einen Alkyl- oder Alkoxyrest mit 1 - 3 Kohlenstoffatomen, ein Halogenatom oder den Trifluormethylrest und

m eine der Zahlen 1 oder 2 bedeuten und deren Säureadditionssalze haben sich als wertvolle Pharmazeutika mit antidepressiven Eigenschaften erwiesen, die insbeson-

./...

EP 0 149 115 A2

dere eine thymoleptische und zentralanregende Wirkung ausüben. Sie können, ausgehend von Oxiranen der allgemeinen Formel IV gemäß Anspruch 1, hergestellt und, z.B. in Form von pharmazeutischen Präparaten, als Heilmittel verwendet werden.

Die Erfindung betrifft neue 2-(1-Imidazolyl)-ethanol-Derivate der allgemeinen Formel

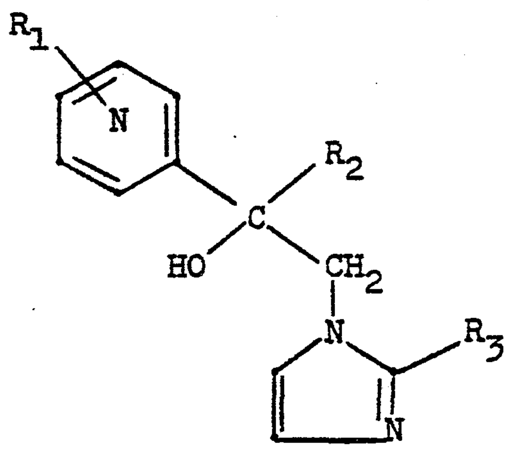

(I)

und deren Säureadditionssalze.

In dieser Formel bedeuten:

$R_1$ Wasserstoff, eine Alkylgruppe mit 1 - 3 Kohlenstoffatomen oder ein Halogenatom;

$R_2$ einen der Reste

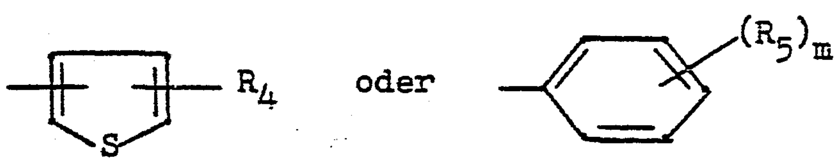

$R_3$ Wasserstoff oder den Methylrest;

$R_4$ Wasserstoff, einen Alkylrest mit 1 - 3 Kohlenstoffatomen oder ein Halogenatom;

$R_5$ Wasserstoff, einen Alkyl- oder Alkoxyrest mit 1 - 3 Kohlenstoffatomen, ein Halogenatom oder den Trifluormethylrest und

m eine der Zahlen 1 oder 2.

Die Erfindung betrifft ferner die Herstellung der neuen Endprodukte und diese Endprodukte enthaltende Arzneimittel, außerdem Zwischenprodukte der allgemeinen Formel IV.

Da die neuen Stoffe der allgemeinen Formel I ein asymmetrisches Kohlenstoffatom besitzen, können sie sowohl als Racemate als auch in Form ihrer Enantiomeren vorliegen.

In bevorzugten Endprodukten der allgemeinen Formel I bedeutet $R_3$ Wasserstoff.
Insbesondere seien genannt die Verbindungen
2-(1-Imidazolyl)-1-(2-pyridyl)-1-(2-thienyl)-ethanol,
2-(1-Imidazolyl)-1-(2-pyridyl)-1-phenyl-ethanol,
2-(1-Imidazolyl)-1-(2-pyridyl)-1-(p-chlor-phenyl)-ethanol,
2-(1-Imidazolyl)-1-(2-pyridyl)-1-(p-brom-phenyl)-ethanol
und 2-(1-Imidazolyl)-1-(2-pyridyl)-1-(p-tolyl)-ethanol
und deren Säureadditionssalze.

Die neuen Stoffe der allgemeinen Formel I und ihre Säureadditionssalze stellen wertvolle Pharmazeutika dar. In einigen spezifischen Tests hat sich herausgestellt, daß sie starke antidepressive Eigenschaften besitzen und insbesondere eine thymoleptische und zentralanregende Wirkung ausüben.

Ein Test zur Bestimmung der antidepressiven Wirkung ist der Tetrabenazin-Antagonismus, die Aufhebung der durch Tetrabenazin verursachten Ptosis. Der Versuch wird an Mäusen durchgeführt, pro Dosis werden 5 Tiere verwendet.

Eine Stunde nach Gabe der Testsubstanz wird den Tieren 40 mg/kg Tetrabenazin verabreicht. Die Beurteilung der Ptosis erfolgt 75 - 120 Minuten nach der Tetrabenazingabe, entsprechend den Angaben von B. Rubin, M.H. Malone et al, J. Pharmacol. Exp. Ther. 120, 125 - 136 (1957).

Bei einem weiteren - biochemischen - Test geht man von der Tatsache aus, daß es bei verschiedenen Depressionsformen in bestimmten Gehirnarealen zu einer Verarmung an biogenen Aminen, vor allem an Noradrenalin, kommt; die biogenen Amine können dadurch vermehrt werden, daß der Uptake in die Neuronen verhindert wird. Eine geeignete Versuchsanordnung zeigt, daß die neuen Verbindungen vor allem die Wiederaufnahme von Noradrenalin in die Neuronen hemmen.
Der Versuch wird, entsprechend den Angaben von A. S. Horn, Mol. Pharmacol. 1971, S. 66 - 80, am homogenisierten isolierten Rattengroßhirn durchgeführt. Eine so erhaltene Synaptosomensuspension wird mit deuteriertem Noradrenalin und verschiedenen Konzentrationen einer Lösung der Testsubstanz in Wasser 10 Minuten bei 37°C inkubiert. Nach Beendigung der Inkubation wird das Medium durch Filtrieren abgetrennt und die Radioaktivität der Synaptosomensuspension gemessen.
Ein Kontrollversuch ohne Teststubstanz läuft zur Bestimmung der Aufnahmemenge der radioaktiven Amine mit.
Als $IC_{50}$ wird diejenige Menge an Testsubstanz in Mol bezeichnet, die ausreicht, um 50 % der Aufnahme (Uptake) zu verhindern.

Die Herstellung der neuen Verbindungen erfolgt in an sich bekannter Weise, z.B.

a) durch Umsetzung von 2-(1-Imidazolyl)-ethanonen der allgemeinen Formel

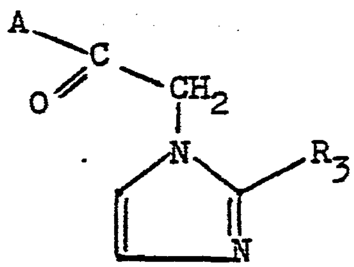

(II)

in der
A einen der Reste

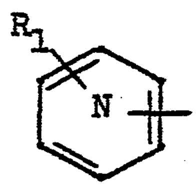

oder $R_2$ bedeutet

und $R_3$ die oben angegebene Bedeutung besitzt, sofern A Pyridyl bedeutet, mit metallorganischen Reagenzien, wie einem Phenyl- oder Thienylmagnesiumhalogenid beziehungsweise, sofern A den Rest $R_2$ bedeutet, einem Pyridylmagnesiumhalogenid, oder einer entsprechenden Lithium-Verbindung, oder

b) durch Reaktion eines Imidazols der allgemeinen Formel

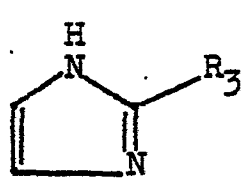

(III)

in der $R_3$ die oben angegebene Bedeutung hat, mit einem Oxiran der allgemeinen Formel

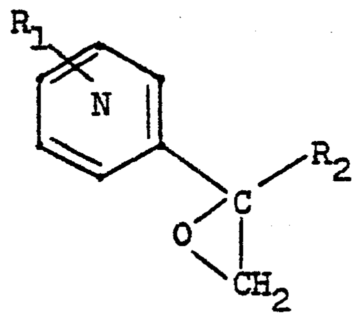

(IV)

in der
$R_1$ und $R_2$ die oben angegebene Bedeutung besitzen.

Bei Verfahren a) werden auf ein Mol der Verbindung II vorzugsweise zwei bis vier Mole des metallorganischen Reagenz eingesetzt. Als Lösungsmittel kommen alle für eine Grignardreaktion üblichen Lösungsmittel oder Lösungsmittelgemische in Frage. Hierzu gehören vorzugsweise Äther, z.B. Diäthyläther, Dimethyläther, Tetrahydrofuran oder Dioxan. Die Reaktionstemperaturen können in einem weiten Bereich variiert werden; kommen Grignardreagenzien zur Anwendung, so arbeitet man bevorzugt bei Temperaturen zwischen 0°C und der Rückflußtemperatur des Lösungsmittels beziehungsweise Lösungsmittelgemisches. Bei Verwendung von Lithium-Verbindungen arbeitet man bevorzugt zwischen 0°C und -60°C.

Die Umsetzung von Verbindungen der allgemeinen Formel III mit Oxiranen IV gemäß Verfahren b) erfolgt im allgemeinen in Gegenwart eines Alkalialkoholats, vorzugsweise der Methylate oder Ethylate von Natrium oder Kalium und in Gegenwart eines Verdünnungsmittels. Als Verdünnungsmittel kommen insbesondere inerte organische Lösungsmittel in Frage. Hierzu gehören z.B. Dimethylformamid, Acetonitril, Methylenchlorid oder aromatische Kohlenwasserstoffe wie Benzol oder Toluol.

Die Reaktionstemperaturen können in einem großen Bereich variiert werden. Im allgemeinen arbeitet man zwischen 10°C und 150°C, vorzugsweise zwischen 30°C und 100°C.
Bei der Durchführung des Verfahrens setzt man auf ein Mol Oxiran der Formel IV vorzugsweise ein bis drei Mol Imidazol beziehungsweise 2-Methylimidazol und ein bis zwei Mol eines Alkalialkoholats ein. Bevorzugt werden die wie nachstehend beschrieben hergestellten Oxirane IV ohne Isolierung mit dem Imidazol beziehungsweise dem 2-Methylimidazol umgesetzt.

Die Isolierung der nach den Verfahren a) und b) erhaltenen Endprodukte der allgemeinen Formel I erfolgt in allgemein üblicher Weise. Sie können gewünschtenfalls nach bekannten Methoden in ihre physiologisch unbedenklichen Säureadditionssalze überführt werden.

Zur Herstellung von Säureadditionssalzen werden insbesondere solche Säuren verwendet, die zur Bildung von therapeutisch verwertbaren Salzen geeignet sind, wie Halogenwasserstoffsäuren, Schwefelsäure, Phosphorsäure, Salpetersäure, Cyclohexylsulfaminsäure, Zitronensäure, Weinsäure, Ascorbinsäure, Maleinsäure, Ameisensäure, Salicylsäure oder Methan- oder Toluolsulfonsäure und dergleichen.

Die Imidazolyl-methyl-arylketone der allgemeinen Formel II sind bekannt beziehungsweise lassen sich in bekannter Weise aus den entsprechenden Arylacylhalogeniden durch Umsetzung mit Imidazol beziehungsweise 2-Methylimidazol in Gegenwart von Verdünnungsmitteln, z.B. Dimethylformamid oder Methylenchlorid, und eines säurebindenden Mittels, insbesondere eines Überschusses an Imidazol, darstellen (vgl. DOS 3101895).

Die Oxirane der allgemeinen Formel IV können in an sich bekannter Weise durch Umsetzung eines entsprechenden Ketons der allgemeinen Formel

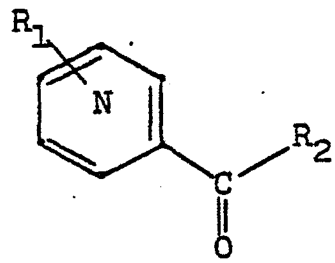

(V)

in der

$R_1$ und $R_2$ die oben angegebene Bedeutung besitzen, entweder mit Dimethyloxosulfoniummethylid der Formel

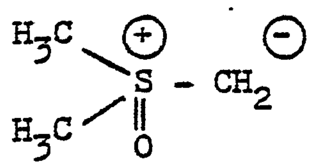

(VI)

oder mit Dimethylsulfoniummethylid der Formel

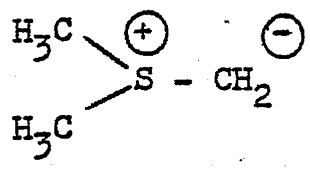

(VII)

in Gegenwart eines Verdünnungsmittels, beispielsweise Dimethylsulfoxid, bei Temperaturen zwischen 20°C und 80°C erhalten werden (vgl. hierzu auch E. J. Corey und M. Chaykovsky, J. Am. Chem. Soc. 87, 1353 (1965) und Heterocycles 8, 397 (1977)).

Die erfindungsgemäßen Verbindungen können in verschiedenen pharmazeutischen Zusammensetzungen angewendet werden.

Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit mischt und in Gelatinekapseln einkapselt.

Geeignete Zäpfchen lassen sich beispielsweise durch Vermischen mit dafür vorgesehen Trägermitteln, wie Neutralfetten oder Polyäthylenglykol beziehungsweise dessen Derivaten, herstellen.

Die nachfolgenden Beispiele erläutern die Erfindung, ohne sie einzuschränken.

Beispiel 1

2-(1-Imidazolyl)-1-(3-pyridyl)-1-phenyl-ethanol

Eine Suspension von 9,35 g (0,05 Mol) 2-(1-Imidazolyl)-1-(3-pyridyl)-ethanon (Fp. 126 - 127°C) in 120 ml Tetrahydrofuran wird bei 50°C zu einer Phenylmagnesiumbromidlösung, erhalten aus 2,4 g (0,1 Mol) Magnesium und 15,7 g (0,1 Mol) Brombenzol in 80 ml Tetrahydrofuran, zugefügt. Das Reaktionsgemisch wird 5 Stunden bei 50°C belassen und nach dem Erkalten mit wäßriger Ammoniumchloridlösung zersetzt. Die organische Phase wird abgetrennt, getrocknet und eingeengt. Der verbleibende Rückstand wird in 2n Salzsäure gelöst. Die sauere wäßrige Lösung wird mit Äther extrahiert und mit konzentriertem Ammoniak alkalisch gestellt. Beim Ausschütteln mit Methylenchlorid scheiden sich weiße Kristalle ab, die abgesaugt werden. Man erhält 5,3 g (40 % d. Th.) 2-(1-Imidazolyl)-1-(3-pyridyl)-1-phenyl-ethanol (Fp. 185 - 187°C). Die analysenreine Verbindung hat einen Schmelzpunkt von 187 - 188°C (aus Acetonitril).

Analog erhält man:

2-(1-Imidazolyl)-1-(3-pyridyl)-1-(p-tolyl)-ethanol, Fp. 181 - 182°C (aus Acetonitril);

2-(1-Imidazolyl)-1-(3-pyridyl)-1-(2-thienyl)-ethanol, Fp. 188 - 188,5°C (aus Acetonitril);

2-(1-Imidazolyl)-1-(2-pyridyl)-1-(p-chlor-phenyl)-ethanol, Fp. 194 - 195°C (aus Ethanol).

Beispiel 2

2-(1-Imidazolyl)-1-(2-pyridyl)-1-(2-thienyl)-ethanol

Eine Lösung von 65,2 g (0,4 Mol) 2-Bromthiophen in 100 ml Äther wird bei -5° - 0°C zu einer Lösung von Butyllithium (250 ml 15%ige Lösung in Hexan, ~ 0,39 Mol) in 300 ml Äther getropft. Nach einstündigem Rühren wird zu der Reaktionsmischung eine Lösung von 30 g (0,16 Mol) 2-(1-Imidazolyl)-1-(2-pyridyl)-ethanon (Fp. 115 - 116°C) in 300 ml Dioxan portionsweise zugefügt. Man entfernt das Kühlbad und läßt noch zwei Stunden nachreagieren. Die Reaktionsmischung wird anschließend mit wäßriger Ammoniumchloridlösung zersetzt. Die anfallenden Kristalle werden abgesaugt und nach dem Trocknen aus Methanol/Petroläther umkristallisiert. Man erhält 19,2 g (44,2 % d.Th.) 2-(1-Imidazolyl)-1-(2-pyridyl)-1-(2-thienyl)-ethanol vom Schmelzpunkt 204 - 205°C. Die analysenreine Base wird mit einem Äquivalent Salzsäure in das Hydrochlorid überführt. Fp. 219 - 220°C (aus Ethanol).

In gleicher Weise wurde hergestellt:

2-(1-Imidazolyl)-1-(2-pyridyl)-1-[2-(5-methyl-thienyl)]-ethanol, Fp. 158 - 159°C (aus Acetonitril).

Beispiel 3

2-(1-Imidazolyl)-1-(2-pyridyl)-1-[2-(5-chlor-thienyl)]-ethanol

Analog Beispiel 2 erhält man durch Umsetzung einer ätherischen Lösung von 5-Chlor-thienyl(2)-Lithium [hergestellt aus 11,8 g (0,1 Mol) 2-Chlorthiophen und 62,5 ml einer 1,6 molaren Lösung von Butyllithium in Hexan bei -50° bis -60°C] und einer Lösung von 9,36 g (0,05 Mol) 2-(1-Imidazolyl)-1-(2-pyridyl)-ethanon in 100 ml Dioxan bei -18° bis -25°C 2-(1-Imidazolyl)-1-(2-pyridyl)-1-[2-(5-chlorthienyl)]-ethanol vom Schmelzpunkt 167 - 168°C (aus Methanol).

Beispiel 4

2-(1-Imidazolyl)-1-(2-pyridyl)-1-phenyl-ethanol

7,2 g Magnesiumspäne werden in 200 ml Äther mit 47,1 g (0,3 Mol) Brombenzol bei 30° bis 35°C zur Reaktion gebracht und anschließend mit einer Lösung von 18,7 g (0,1 Mol) 2-(1-Imidazolyl)-1-(2-pyridyl)-ethanon in 200 ml Dioxan tropfenweise versetzt. Die Reaktionsmischung wird 4 Stunden unter Rückfluß erhitzt und nach dem Abkühlen mit wäßriger Ammoniumchloridlösung zersetzt. Die anfallenden Kristalle werden abgetrennt und mit verdünnter Salzsäure aufgenommen. Anschließend wird die wäßrige Lösung mit Methylenchlorid ausgeschüttelt, mit konzentriertem Ammoniak alkalisch gestellt und mit Methylenchlorid extrahiert. Die organische Phase wird über Natriumsulfat getrocknet und eingeengt. Man erhält 14,7 g (55,4 % d.Th.) 2-(1-Imidazolyl)-1-(2-pyridyl)-1-phenyl-ethanol vom Schmelzpunkt 175 - 177°C.

Die reine Titelverbindung hat nach dem Umkristallisieren aus Alkohol einen Schmelzpunkt von 177 - 178°C.

Analog wurden erhalten:

2-(1-Imidazolyl)-1-(2-pyridyl)-1-(p-tolyl)-ethanol, Fp. 195 - 196°C (aus Ethanol);

2-(1-Imidazolyl)-1-(2-pyridyl)-1-(p-methoxy-phenyl)-ethanol, Fp. 154 - 156°C (aus Acetonitril);

2-(1-Imidazolyl)-1-(3-pyridyl)-1-(p-chlorphenyl)-ethanol, Fp. 176 - 178°C (aus Methanol/Äther);

2-(2-Methyl-1-imidazolyl)-1-(2-pyridyl)-1-(p-chlorphenyl)-ethanol, Fp. 209 - 210°C (aus Ethanol);

2-(1-Imidazolyl)-1-(2-pyridyl)-1-(p-bromphenyl)-ethanol, Fp. 193 - 194°C (aus Ethanol).

Beispiel 5

2-(1-Imidazolyl)-1-(2-pyridyl)-1-phenyl-ethanol

Eine Lösung von 4,7 g (0,024 Mol) 2-Phenyl-2-(2-pyridyl)-oxiran in 30 ml Dimethylformamid wird zu einer Mischung aus 2,2 g (0,04 Mol) Natriummethylat, 15 ml Methanol und 4,5 g (0,066 Mol) Imidazol getropft. Das Reaktionsgemisch wird 4 Stunden auf 80°C erhitzt und anschließend im Vakuum eingedampft. Der Rückstand wird zwischen Chloroform und Wasser verteilt. Die organische Phase wird abgetrennt, über Natriumsulfat getrocknet und eingeengt. Die anfallenden Kristalle werden mit Äther suspendiert, abgesaugt und aus Toluol umkristallisiert.

Man erhält 4,8 g (76 % d.Th.) 2-(1-Imidazolyl)-1-(2-pyridyl)-1-phenyl-ethanol vom Schmelzpunkt 176 - 178°C (s. auch Beispiel 4).

Das als Ausgangsstoff verwendete 2-Phenyl-2-(2-pyridyl)-oxiran wurde wie folgt erhalten:

Zu einer Suspension von 2,56 g (0,053 Mol) Natriumhydrid (~50%ig in Öl) in 50 ml Dimethylsulfoxid wird unter Rühren eine Lösung von 7,1 g (0,039 Mol) 2-Benzoylpyridin in 100 ml Dimethylsulfoxid getropft. Anschließend werden 12 g (0,06 Mol) Trimethylsulfoniumjodid hinzugefügt. Die erhaltene Reaktionsmischung wird 4 Stunden nachgerührt, vorsichtig mit 300 ml Wasser versetzt und mit Äther extrahiert. Die organische Phase wird mit Wasser gewaschen und nach dem Trocknen über Natriumsulfat eingeengt. Der Rückstand (7,5 g) wird in wenig Chloroform gelöst und auf eine Kieselgelsäule aufgebracht. Nach dem Abtrennen einer Verunreinigung mit Petroläther (40° - 60°C) wird das Hauptreaktionsprodukt mit einem Chloroform-Essigestergemisch (8:2) eluiert. Man erhält 6 g (78 % d.Th.) 2-Phenyl-2-(2-pyridyl)-oxiran als Öl mit folgenden Kenndaten:

<u>NMR-Daten*</u> 8,60 ppm (1H, m, Pyridyl/6' Pos.); 7,11 - 7,77 (8H, m, Pyridyl/3',4',5' Pos. und Phenyl); 3,53 ppm (1H, d, J=6Hz, Oxiran); 3,22 ppm (1H, d, J=6Hz, Oxiran)

Ausgehend von 4-Benzoyl-pyridin erhält man in analoger Weise:
2-Phenyl-2-(4-pyridyl)-oxiran:

NMR-Daten* 8,55 ppm (2H, d, J=6Hz, Pyridyl/2',6' Pos.); 7,23 ppm (2H, d, J=6Hz, Pyridyl/3', 5' Pos.); 7,36 ppm (5H, s, Phenyl); 3,37 ppm (1H, d, J=6Hz, Oxiran); 3,15 ppm (1H, d, J=6Hz, Oxiran)

Analog wurden hergestellt:

2-(4-Chlorphenyl)-2-(2-pyridyl)-oxiran

2-(4-Bromphenyl)-2-(2-pyridyl)-oxiran

2-(2-Pyridyl)-2-(2-thienyl)-oxiran

2-(3,4-Dichlorphenyl)-2-(2-pyridyl)-oxiran

2-(4-Methoxyphenyl)-2-(3-pyridyl)-oxiran

2-Phenyl-2-[5-chlor-pyridyl(2)]-oxiran

* Die $^{1}$H-NMR-Spektren wurden mit einem Bruker WH 90-Gerät aufgenommen (s = Singulett, d = Dublett und m = Multiplett).

Meßbedingungen:

$^{1}$H-Frequenz: 90 MHz
Temperatur: 25°C; Lösungsmittel: $CDCl_3$
Spektrale Breite: 1160 Hz = 12,88 ppm
Normierung: Internes Tetramethylsilan = 0 ppm als Bezugspunkt für die chemischen Verschiebungen (Signalpositionen) im Spektrum.

Beispiel 6

2-(1-Imidazolyl)-1-(4-pyridyl)-1-phenyl-ethanol-fumarat

Ausgehend von 4,7 g (0,024 Mol) 2-Phenyl-2-(4-pyridyl)-oxiran erhält man analog Beispiel 5 2-(1-Imidazolyl)-1-(4-pyridyl)-1-phenyl-ethanol.

Zur Darstellung des fumarsauren Salzes wird die Base mit einer äquivalenten Menge Fumarsäure unter Erwärmen in Methanol gelöst. Die beim Abkühlen der Lösung anfallenden Kristalle werden aus Methanol umkristallisiert. Ausbeute: 6,5 g (71,4 % d. Th.); Fp. 201°C (Zers.).

Für die neuen Verbindungen beziehungsweise ihre Säureadditionssalze wird eine Dosierung von 5 - 50, vorzugsweise 10 - 30 mg pro Dosis, vorgeschlagen.

## Formulierungsbeispiele

a) Dragees

1 Drageekern enthält:

| | |
|---|---|
| Wirkstoff gemäß der Erfindung | 25,0 mg |
| Milchzucker | 50,0 mg |
| Maisstärke | 22,0 mg |
| Gelatine | 2,0 mg |
| Magnesiumstearat | 1,0 mg |
| | 100,0 mg |

Herstellung:

Die Mischung der Wirksubstanz mit Milchzucker und Maisstärke wird mit einer 10%igen wäßrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxyd, Talkum und Gummi arabicum aufgebracht wird. Die fertigen Dragees werden mit Bienenwachs poliert.
Dragee-Endgewicht: 200 mg

b) Tabletten

| | |
|---|---|
| Wirkstoff gemäß der Erfindung | 10,0 mg |
| Milchzucker | 40,0 mg |
| Maisstärke | 44,0 mg |
| lösliche Stärke | 5,0 mg |
| Magnesiumstearat | 1,0 mg |
| | 100,0 mg |

Herstellung:

Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die je 10 mg Wirkstoff enthalten.

c) Suppositorien

1 Zäpfchen enthält:

| | |
|---|---|
| Wirkstoff gemäß der Erfindung | 10,0 mg |
| Zäpfchenmasse | 1.690,0 mg |

Herstellung:

Die feingepulverte Substanz wird mit Hilfe eines Eintauch-Homogenisators in die geschmolzene und auf 40°C abgekühlte Zäpfchenmasse eingerührt. Die Masse wird bei 35°C in leicht vorgekühlte Formen gegossen.

d) Ampullen (Injektionslösungen)

Zusammensetzung:

| | |
|---|---|
| Wirkstoff gemäß der Erfindung | 5,0 Gew.-Teile |
| Natriumpyrosulfit | 1,0 Gew.-Teile |
| Dinatriumsalz der Äthylendiamintetraessigsäure | 0,5 Gew.-Teile |
| Natriumchlorid | 8,5 Gew.-Teile |
| doppelt destilliertes Wasser ad | 1000,0 Gew.-Teile |

Herstellung:

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 5,0 mg Wirkstoff.

**P a t e n t a n s p r ü c h e .**

1. Neue 2-(1-Imidazolyl)-ethanol-Derivate der allgemeinen Formel

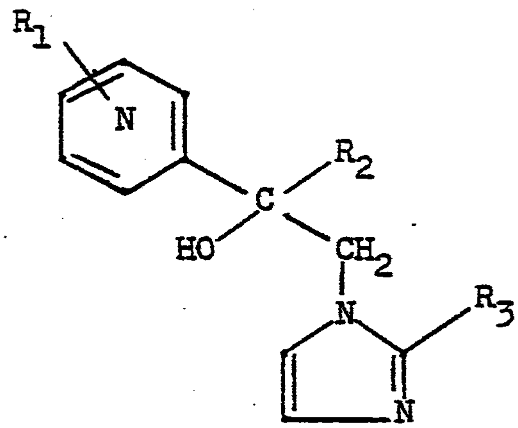

(I)

worin

$R_1$ Wasserstoff, einen Alkylrest mit 1 - 3 Kohlenstoffatomen oder ein Halogenatom,

$R_2$ einen der Reste

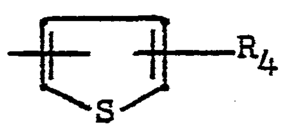

oder

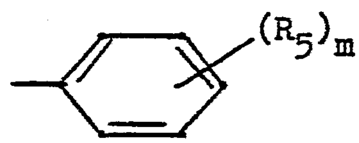

$R_3$ Wasserstoff oder den Methylrest,

$R_4$ Wasserstoff, einen Alkylrest mit 1 - 3 Kohlenstoffatomen oder ein Halogenatom,

$R_5$ Wasserstoff, einen Alkyl- oder Alkoxyrest mit 1 - 3 Kohlenstoffatomen, ein Halogenatom oder die Trifluormethylgruppe und

m eine der Zahlen 1 oder 2 bedeuten

und deren physiologisch verträgliche Säureadditionssalze.

2. Neue Verbindungen der allgemeinen Formel I gemäß Anspruch 1 sowie deren physiologisch verträgliche Säureadditionssalze, dadurch gekennzeichnet, daß der Rest $R_3$ für Wasserstoff steht, während die übrigen Reste die in Anspruch 1 angegebene Bedeutung besitzen.

3. 2-(1-Imidazolyl)-1-(2-pyridyl)-1-(2-thienyl)-ethanol und dessen physiologisch verträgliche Säureadditionssalze.

4. 2-(1-Imidazolyl)-1-(2-pyridyl)-1-[2-(5-chlor-thienyl)]-ethanol und dessen physiologisch verträgliche Säureadditionssalze.

5. 2-(1-Imidazolyl)-1-(2-pyridyl)-1-(p-chlor-phenyl)-ethanol und dessen physiologisch verträgliche Säureadditionssalze.

6. 2-(1-Imidazolyl)-1-(2-pyridyl)-1-(p-brom-phenyl)-ethanol und dessen physiologisch verträgliche Säureadditionssalze.

7. 2-(1-Imidazolyl)-1-(2-pyridyl)-1-(p-tolyl)-ethanol und dessen physiologisch verträgliche Säureadditionssalze.

8. Verfahren zur Herstellung neuer 2-(1-Imidazolyl)-ethanol-Derivate der allgemeinen Formel

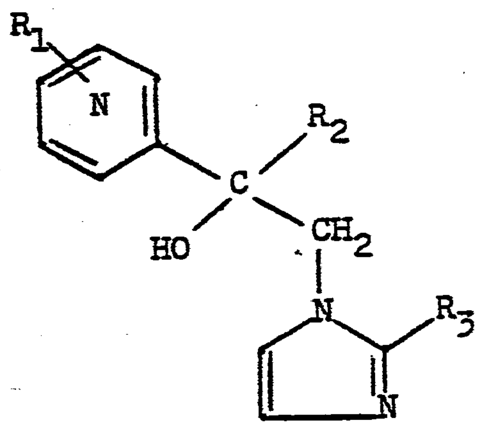

(I)

worin

$R_1$ Wasserstoff, einen Alkylrest mit 1 - 3 Kohlenstoffatomen oder ein Halogenatom,

$R_2$ einen der Reste

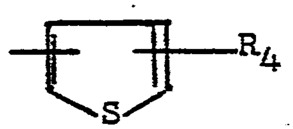

oder

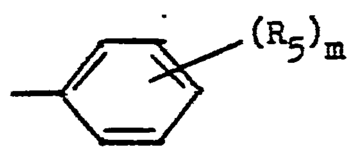

$R_3$ Wasserstoff oder den Methylrest,

$R_4$ Wasserstoff, einen Alkylrest mit 1 - 3 Kohlenstoffatomen oder ein Halogenatom,

$R_5$ Wasserstoff, einen Alkyl- oder Alkoxyrest mit 1 - 3 Kohlenstoffatomen, ein Halogenatom oder die Trifluormethylgruppe und

m eine der Zahlen 1 oder 2 bedeuten sowie von deren physiologisch verträglichen Säureadditionssalzen, dadurch gekennzeichnet, daß man

a) ein 2-(1-Imidazolyl)-ethanon der allgemeinen Formel

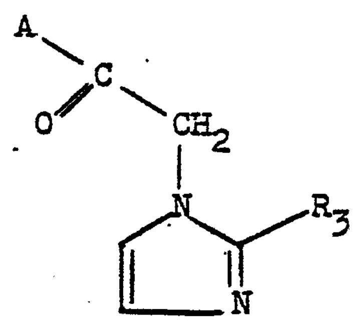

(II)

worin

A einen der Reste

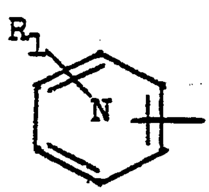 oder $R_2$ bedeutet und

$R_3$ die angegebene Bedeutung besitzt, sofern A Pyridyl bedeutet, mit metallorganischen Reagenzien, wie einem Phenyl- oder Thienylmagnesiumhalogenid beziehungsweise, sofern A den Rest $R_2$ bedeutet, einem Pyridylmagnesiumhalogenid oder einer entsprechenden Lithium-Verbindung umsetzt, oder daß man

b) ein Imidazol der allgemeinen Formel

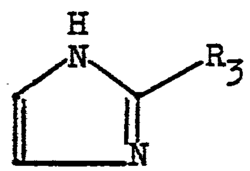

(III)

worin $R_3$ die oben angegebene Bedeutung besitzt, mit einem Oxiran der allgemeinen Formel

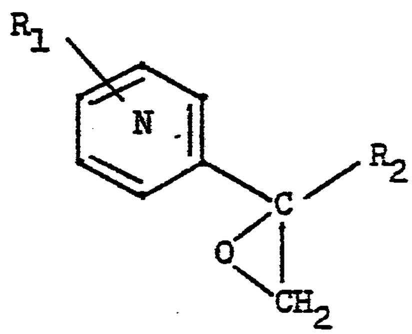

(IV)

worin
$R_1$ und $R_2$ die oben angegebene Bedeutung besitzen, zur Reaktion bringt, und daß man gegebenenfalls in an sich bekannter Weise ein so erhaltenes Endprodukt der allgemeinen Formel I in ein physiologisch unbedenkliches Säureadditionssalz überführt.

9. Pharmazeutische Präparate, enthaltend als Wirkstoffe Verbindungen der allgemeinen Formel I in Kombination mit üblichen Hilfs- und/oder Trägerstoffen.

10. Verfahren zur Herstellung von pharmazeutischen Präparaten nach Anspruch 9 , dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

11. Verbindungen der allgemeinen Formel I gemäß Anspruch 1 zur Verwendung für die Herstellung von Arzneimitteln mit antidepressiver Wirkung.

12. Methode zur Behandlung von Depressionen mittels pharmazeutischer Präparate gemäß Anspruch 9.